# EUROPEAN PATENT APPLICATION

(11) **EP 4 609 868 A1**
(43) Date of publication of application: **03.09.2025**
(21) Application number: 23882546.7
(22) Date of filing: 20.10.2023
(51) Int. Cl.: A61K 31/737, A23L 33/105, A61K 36/05, A61P 39/06, A61P 43/00

(54) **ANTI-AGING AGENT**

(30) Priority: 25.10.2022 JP 2022170789
(71) Applicant: National University Corporation Kochi University, Kochi-shi, Kochi 780-8520 (JP)
(72) Inventor: ONDA, Ayumu, Kochi-shi, Kochi 780-8520 (JP); NAMBA, Takushi, Kochi-shi, Kochi 780-8520 (JP)
(74) Representative: Müller-Boré & Partner Patentanwälte PartG mbB
(86) International application number: PCT/JP2023/037984
(87) International publication number: WO 2024/090341

(57) **Abstract**

The objective of the present invention is to provide an anti-aging agent having excellent actions such as a mitochondria activating action. The anti-aging agent according to the present invention is characterized in comprising H-type ulvan or an alkali metal ion salt of ulvan as an active ingredient.

## Description

### TECHNICAL FIELD

The present invention relates to an anti-aging agent having excellent actions such as a mitochondria activating action.

### BACKGROUND ART

The average life expectancy in Japan significantly increased in the second half of the 20th century, and thus Japan has become one of the world's leading countries in terms of life expectancy. On the one hand, the problems caused by aging are becoming more common. For example, aging becomes evident as a cognitive decline, a decline of muscle strength, osteoporosis, increase in visceral fat, a decline in cardiovascular function and insomnia.

In the past, aging process was thought to be extremely complex and impossible to treat. On the one hand, the advance in science have recently revealed that aging may be treated as one of cellular biological processes, and anti-aging technology has been studied in order to treat a biological process of aging, lower the incidence rate of age-related diseases such as arteriosclerosis and cancer caused by aging, and aim for a long and healthy life with keeping QOL: Quality Of Life.

Some algae produce and accumulate large amounts of polysaccharides through photosynthesis. For example, alginate produced by brown algae has been utilized in the food field, the medical field and the industrial field as a thickener, a stabilizing agent, a gelator, a dietary fiber material or the like, since alginate becomes gelled by a divalent metal ion such as calcium ion and magnesium ion.

For example, Ulva meridionalis has been expected to be utilized as a biomass resource, since Ulva meridionalis is a large green alga classified in Ulva and has one of the highest growth rates of all plants. Ulva meridionalis contains about 20 to 30% of ulvan as a polysaccharide in addition to about 5 to 15% of starch and cellulose per dry weight of the alga body.

For example, ulvan was investigated for use in combination with hyaluronic acid to treat the signs of skin aging and to treat and heal skin wounds (Patent document 1).

### PRIOR ART DOCUMENT

### PATENT DOCUMENT

Patent document 1: JP 2017-514825 T

### DISCLOSURE OF THE INVENTION

### PROBLEMS TO BE SOLVED BY THE INVENTION

It has been investigated as described above to combine ulvan obtained from algae with hyaluronic acid to treat the signs of skin aging or the like (Patent document 1).

Patent document 1, however, merely discloses the experimental data to show that the combination of Ascophyscient (registered trademark), which is a fucoidan-rich crude extract product derived from Ascophyllum nodosum as chrysophyte, and hyaluronic acid significantly accelerates the growth of a fibroblast cell. Though a fibroblast cell is a cell that constitutes a connective tissue and produces dermal components such as collagen, elastin and hyaluronic acid, it is unclear whether a compound to accelerate the growth of a fibroblast cell can actually improve skin fitness or not, and it is also unclear whether such a component can retard general senescence or not.

The objective of the present invention is to provide an anti-aging agent having excellent actions such as a mitochondria activating action.

### MEANS FOR SOLVING THE PROBLEMS

The inventors of the present invention made extensive studies to solve the above-described problems. As a result, the inventors completed the present invention by finding that the specific derivatives of natural ulvan has significantly excellent anti-aging actions.

Hereinafter, the present invention is described.
[1] An anti-aging agent comprising an H-type ulvan or an alkali metal ion salt of ulvan as an active ingredient.
[2] The anti-aging agent according to the above [1], wherein the alkali metal is Na and/or K.
[3] The anti-aging agent according to the above [1], wherein 15 mol% or more of a carboxy group and a sulfate group of the H-type ulvan is protonated.
[4] The anti-aging agent according to the above [1] or [2], wherein 30 mol% or more of a carboxy group and a sulfate group of the alkali metal ion salt of ulvan form the salt of the alkali metal ion.
[5] The anti-aging agent according to any one of the above [1] to [4], having a mitochondria activating action.
[6] The anti-aging agent according to any one of the above [1] to [4], having an antioxidant action.
[7] The anti-aging agent according to any one of the above [1] to [4], having a collagen biosynthesis enhancing action.
[8] Use of an H-type ulvan or an alkali metal ion salt of ulvan for retarding senescence.
[9] The use according to the above [8], wherein the alkali metal is Na and/or K.
[10] The use according to the above [8], wherein 15 mol% or more of a carboxy group and a sulfate group of the H-type ulvan is protonated.
[11] The use according to the above [8] or [9], wherein 30 mol% or more of a carboxy group and a sulfate group of the alkali metal ion salt of ulvan form the salt of the alkali metal ion.
[12] The use according to any one of the above [8] to [11], wherein senescence is retarded by activating mitochondria.
[13] The use according to any one of the above [8] to [11], wherein senescence is retarded by an antioxidant action.
[14] The use according to any one of the above [8] to [11], wherein senescence is retarded by enhancing collagen biosynthesis.
[15] A method for retarding senescence of an animal, comprising the step of administering an H-type ulvan or an alkali metal ion salt of ulvan to the animal.
[16] The method according to the above [15], wherein the alkali metal is Na and/or K.
[17] The method according to the above [15], wherein 15 mol% or more of a carboxy group and a sulfate group of the H-type ulvan is protonated.
[18] The method according to the above [15] or [16], wherein 30 mol% or more of a carboxy group and a sulfate group of the alkali metal ion salt of ulvan form the salt of the alkali metal ion.
[19] The method according to any one of the above [15] to [18], wherein the animal is a human.
[20] The method according to any one of the above [15] to [18], wherein the animal is an animal other than a human.
[21] The method according to any one of the above [15] to [18], wherein senescence is retarded by activating mitochondria.
[22] The method according to any one of the above [15] to [18], wherein senescence is retarded by an antioxidant action.
[23] The method according to any one of the above [15] to [18], wherein senescence is retarded by enhancing collagen biosynthesis.
[24] Use of an H-type ulvan or an alkali metal ion salt of ulvan for producing a medical agent for retarding senescence.
[25] The use according to the above [24], wherein the alkali metal is Na and/or K.
[26] The use according to the above [24], wherein 15 mol% or more of a carboxy group and a sulfate group of the H-type ulvan is protonated.
[27] The use according to the above [24] or [25], wherein 30 mol% or more of a carboxy group and a sulfate group of the alkali metal ion salt of ulvan form the salt of the alkali metal ion.
[28] The use according to any one of the above [24] to [27], wherein senescence is retarded by activating mitochondria.
[29] The use according to any one of the above [24] to [27], wherein senescence is retarded by an antioxidant action.
[30] The use according to any one of the above [24] to [27], wherein senescence is retarded by enhancing collagen biosynthesis.
[31] An H-type ulvan or an alkali metal ion salt of ulvan for retarding senescence.
[32] The H-type ulvan or the alkali metal ion salt of ulvan according to the above [31], wherein the alkali metal is Na and/or K.
[33] The H-type ulvan or the alkali metal ion salt of ulvan according to the above [31], wherein 15 mol% or more of a carboxy group and a sulfate group of the H-type ulvan is protonated.
[34] The H-type ulvan or the alkali metal ion salt of ulvan according to the above [31] or [32], wherein 30 mol% or more of a carboxy group and a sulfate group of the alkali metal ion salt of ulvan form the salt of the alkali metal ion.
[35] The H-type ulvan or the alkali metal ion salt of ulvan according to any one of the above [31] to [34], wherein senescence is retarded by activating mitochondria.
[36] The H-type ulvan or the alkali metal ion salt of ulvan according to any one of the above [31] to [34], wherein senescence is retarded by an antioxidant action.
[37] The H-type ulvan or the alkali metal ion salt of ulvan according to any one of the above [31] to [34], wherein senescence is retarded by enhancing collagen biosynthesis.

### EFFECT OF THE INVENTION

The anti-aging agent of the present invention has significant and excellent anti-aging actions such as reduction in an aging marker, acceleration of the production of collagen, acceleration of the expression of an antioxidant enzyme and activation of mitochondria. In addition, the ulvan derivatives as the active ingredient of the anti-aging agent according to the present invention are polysaccharides formed by polymerizing glucuronic acid, sulfated rhamnose or the like and may be highly safe. Thus, the present invention is industrially very excellent as a technology associated with an excellent anti-aging agent.

### BRIEF DESCRIPTION OF THE DRAWINGS

[Figure 1] Figure 1 are enlarged photographs of human skin fibroblast cells of which senescence beta galactosidase as a senescence marker was stained and that were cultivated in the culture media containing (1) distilled water only (control), (2) natural ulvan, (3) H-type ulvan, (4) ulvan 41 mol% alkali metal ion (AMI) salt and (5) ulvan 93 mol% AMI salt.
[Figure 2] Figure 2 is a graph to show the proportions of the human skin fibroblast cells containing a senescence beta galactosidase as a senescence marker per 50 human skin fibroblast cells cultivated in the culture media containing distilled water only (control), natural ulvan, H-type ulvan or ulvan AMI salt.
[Figure 3] Figure 3 is a graph to show the relative amounts of the collagen produced by the human skin fibroblast cell cultivated in the culture media containing distilled water only (control), natural ulvan, H-type ulvan or ulvan AMI salt.
[Figure 4] Figure 4 are photographs of the gels showing the bands of β actin and mitochondria SOD2 by polyacrylamide electrophoresis for analyzing the proteins contained in the human skin fibroblast cell cultivated in the culture media containing distilled water only (control), natural ulvan, H-type ulvan or ulvan AMI salt.
[Figure 5] Figure 5 are photographs of the gels showing the bands of serine/threonine kinase (Total-AKT) and phosphorylated AKT (P-AKT) by polyacrylamide electrophoresis for analyzing the proteins contained in the human skin fibroblast cell cultivated in the culture media containing natural ulvan or H-type ulvan.
[Figure 6] Figure 6 is a graph to show the mitochondria membrane potentials corresponding to the mitochondria activities of the human skin fibroblast cell cultivated in the culture media containing distilled water only (control), natural ulvan, H-type ulvan or ulvan AMI salt.
[Figure 7] Figure 7 is a graph to show the ATP amounts produced by the human skin fibroblast cell cultivated in the culture media containing distilled water only (control), natural ulvan, H-type ulvan or ulvan AMI salt.
[Figure 8] Figure 8 is a graph to show the amounts of antioxidant enzyme SOD produced by the human skin fibroblast cell cultivated in the culture media containing distilled water only (control), natural ulvan or H-type ulvan.
[Figure 9] Figure 9 is a graph to show the relative amounts of mRNA of Filaggrin and antioxidant enzyme SOD2 of mitochondria as epidermal protective factors produced by the human skin fibroblast cell cultivated in the culture media containing distilled water only (control), natural ulvan, H-type ulvan or ulvan AMI salt.
[Figure 10] Figure 10 is a graph to show the relative amounts of antioxidant enzyme SOD2 of mitochondria produced by the human skin fibroblast cell cultivated in the culture media containing distilled water only (control), natural ulvan, H-type ulvan or ulvan AMI salt.
[Figure 11] Figure 11 is a graph to show the relative amounts of antioxidant enzyme SOD2 of mitochondria produced by the human skin fibroblast cell cultivated in the culture media containing distilled water only (control), natural ulvan or K-type ulvan AMI salt.

### MODE FOR CARRYING OUT THE INVENTION

The anti-aging agent of the present invention comprises an H-type ulvan or an alkali metal salt of ulvan as an active ingredient.

Ulvan is a polysaccharide produced by green algae classified in Enteromorpha sp., such as Ulva meridionalis, and green algae classified in Ulva sp., such as Ulva ohnoi. Ulvan is a sulfated anionic polysaccharide containing uronic acid such as glucuronic acid and iduronic acid, and rhamnose, galactose, xylose, glucose or the like that have a sulfate group. Uronic acid and sulfated rhamnose or the like are alternately polymerized in about 2/3 to 3/4 part of ulvan, and sulfated rhamnose or the like is mainly polymerized in the other part. The naturally derived ulvan is hereinafter referred to as natural ulvan in some cases.

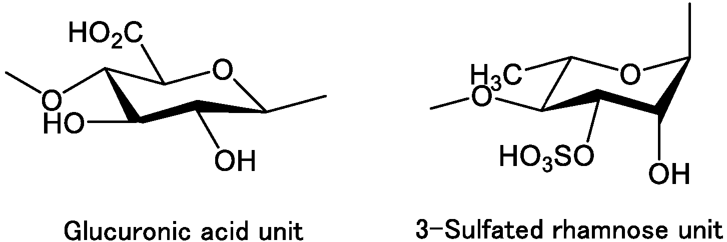

Natural ulvan mainly has the following two structural units:
· ulvanobiuronic acid 3-sulfate-type A formed by binding β-D-glucuronic acid and 3-sulfated α-L-rhamnose through 1→4 bond;
· ulvanobiuronic acid 3-sulfate-type B formed by binding α-L-iduronic acid and 3-sulfated α-L-rhamnose through 1→4 bond.

Natural ulvan mainly contains a magnesium ion, a calcium ion, a sodium ion and a potassium ion, and contains a magnesium ion most as the cation. Ulvan is less likely to be gelled in comparison with alginic acid or the like. Even when ulvan contains large amount of a magnesium ion, the sol state of ulvan is maintained in many cases.

Natural ulvan can be extracted from algae containing ulvan, such as Ulva meridionalis, by an ordinary method. For example, a raw material alga is pulverized and then water or the like may be added as a solvent for extraction at about 80°C higher and 120°C lower, since ulvan is water-soluble. Since ulvan is insoluble in a general organic solvent, ulvan is precipitated by adding a water-miscible organic solvent such as methanol, ethanol and 2-propanol to the extraction liquid. The precipitated ulvan may be separated from the solvent by filtration and centrifugation, and dried.

The H-type ulvan as the active ingredient of the anti-aging agent according to the present invention means the ulvan of which at least a part of carboxy group and sulfate group are protonated. In other words, the carboxy group and the sulfate group are in a state of -CO₂H and -OSO₃H. The carboxy group and the sulfate group of ulvan are hereinafter referred to as an acidic group together in some cases. Specifically, the H-type ulvan means the ulvan of which ratio of the total molar numbers of the protonated carboxy group (-CO₂H) and the protonated sulfate group (-OSO₃H) to the molar number of the total acidic group is 10 mol% or more. The ratio is preferably 15 mol% or more, more preferably 19 mol% or more, 20 mol% or more or 30 mol% or more, and even more preferably 35 mol% or more or 40 mol% or more. The upper limit of the ratio is not particularly restricted and may be 100 mol%. Since it is sometimes difficult to protonate all of the carboxy group and the sulfate group in natural ulvan, the ratio is preferably 95 mol% or less or 90 mol% or less and more preferably 80 mol% or less or 70 mol% or less. A sulfate group is a stronger acid than a carboxy group. For example, the Ka of sulfuric acid is 10^{6.75} times of that of formic acid as the acid dissociation constant pKa of formic acid is 3.75 and that of sulfuric acid is -3.0. Thus, the carboxy group may be protonated more preferentially than sulfate group in the H-type ulvan. For example, the proportion of a protonated acidic group can be determined by measuring the amount of the cation in a ulvan sample with inductively coupled plasma (ICP) emission spectrometric analysis and estimating the amount of the proton on the basis of the measured data on the presumption that all of the cation and proton exist in the acidic group.

The alkali metal ion salt of ulvan as the active ingredient of the anti-aging agent according to the present invention means the ulvan of which at least a part of the divalent metal ion such as calcium ion and magnesium ion forming a salt with the carboxy group or the sulfate group of natural ulvan is substituted with an alkali metal ion. Specifically, the alkali metal ion salt of ulvan means the ulvan of which ratio of the molar number of the divalent ion to the molar number of the total acidic group is 30 mol% or less and the molar ratio of the alkali metal ion is 20 mol% or more. The ratio of the divalent ion is preferably 20 mol% or less and more preferably 10 mol% or less. The ratio of the alkali metal ion is preferably 30 mol% or more and more preferably 40 mol% or more. The upper limit of the ratio is not particularly restricted and may be 100 mol%. Since it is sometimes difficult to convert all of the acidic group in natural ulvan into alkali metal ion salts, the ratio is preferably 95 mol% or less or 90 mol% or less and more preferably 80 mol% or less. For example, the ratios of the molar numbers of the proton and alkali metal ion to the molar number of the total acidic groups in the H-type ulvan and the alkali metal ion salt of ulvan of the present invention can be determined by inductively coupled plasma (ICP) emission spectrometric analysis. Though C, O, N and H in the air and water in a sample cannot be determined by ICP emission spectrometric analysis, the molar numbers of the total acidic group and the proton in the objective ulvan sample can be estimated by the following procedure and the ratios of the proton and the alkali metal ion to the acidic group can be calculated: (1) the objective ulvan sample is sufficiently washed to remove free cation, (2) the washed objective ulvan sample is treated with an ion-exchange resin or the like to convert all of the acidic group into a metal ion salt, (3) the washed objective ulvan sample and the metal ion-salificated objective ulvan sample are analyzed by ICP spectrometry, and (4) the both of the analytical data are compared.

Even when the active ingredient of the present invention is the H-type ulvan, a part of the acidic group may be an alkali metal salt and/or an alkaline-earth metal salt, and even when the active ingredient is an alkali metal ion salt of ulvan, a part of the acidic group may be protonated. In other words, the active ingredient of the present invention may be said to be both of the H-type ulvan and the alkali metal salt of which acidic group is sufficiently protonated and converted into an alkali metal-salt.

An example of the alkali metal ion includes lithium ion, sodium ion, potassium ion, rubidium ion and cesium ion. The alkali metal ion is preferably 1 or more alkali metal ions selected from lithium ion, sodium ion and potassium ion, more preferably sodium ion and/or potassium ion, and more preferably sodium ion.

The molecular weight of the H-type ulvan and the alkali metal ion salt of ulvan as the active ingredient of the anti-aging agent according to the present invention is not particularly restricted and for example, may be 10,000 or more. The molecular weight is preferably 100,000 or more, more preferably 200,000 or more and even more preferably 500,000 or more. The upper limit of the molecular weight is not particularly restricted, but since the operatability thereof may be possibly difficult in the case where the molecular weight is excessively large, the molecular weight is preferably 2,000,000 or less, more preferably 1,500,000 or less and even more preferably 1,000,000 or less.

The H-type ulvan and the alkali metal ion salt of ulvan can be produced by substituting the cation in natural ulvan with a proton (H⁺) or an alkali metal ion as an ordinary method. For example, an OH-type anion-exchange resin is added to an aqueous solution of natural ulvan, the mixture is treated at 0°C or higher and 100°C or lower for 10 minutes or more and 1 hour or less, and then the anion-exchange resin is removed by filtration or centrifugation to adsorb and remove the anion such as sulfate ion and chloride ion contained in natural ulvan. Then, the alkali metal ion salt of ulvan can be produced by adding an alkali ion-type ion-exchange resin to the ulvan solution to be treated at 0°C or higher and 100°C or lower for 10 minutes or more and 1 hour or less and then removing the cation-exchange resin with filtration or centrifugation. In addition, the H-type ulvan can be produced by adding an H-type cation-exchange resin to an aqueous solution of the alkali metal ion salt of ulvan to be treated at 0°C or higher and 100°C or lower for 10 minutes or more and 1 hour or less and then removing the cation-exchange resin with filtration or centrifugation. The H-type ulvan and the alkali metal ion salt of ulvan can be isolated by freeze dry or the like from the aqueous solution of the H-type ulvan and the alkali metal ion salt of ulvan. The H-type ulvan and the alkali metal ion salt of ulvan are hereinafter referred to as the ulvan derivative together in some cases.

The inventors of the present invention experimentally found that the ulvan derivative of the present invention activates mitochondria. Mitochondria metabolizes a carbohydrate, a protein, a lipid or the like to produce an energy substance such as ATP and heat. Thus, the ulvan derivative of the present invention not only contributes to the energy supply to maintain homeostatic life activity but also may accelerate the secretion of 4-aminobutyric acid (GABA) with tranquilizing property and insulin to lower blood glucose level.

The inventors of the present invention also experimentally found that the ulvan derivative of the present invention accelerates the gene expression and the biosynthesis of an antioxidant enzyme of mitochondria. An active oxygen in a living body plays an important role to protect the living body by attacking bacteria and viruses invading the living body under normal conditions, but excessive active oxygen also attacks a normal cell and may cause a vicious cycle that the lipid peroxide produced by active oxygen further produces active oxygen in some cases. For example, the ulvan derivative of the present invention not only activates mitochondria through the activation of an antioxidant enzyme of mitochondria but also may contribute the maintenance of health and the maintenance of good skin condition, since the antioxidant enzyme decomposes excessive active oxygen to be detoxified.

In addition, the inventors of the present invention experimentally demonstrated that the ulvan derivative of the present invention accelerates the biosynthesis of collagen. It has been known that collagen is a fibrous protein constituting skin, tendon, cartilage or the like, and aging causes the wrinkles of the skin and the decay of skin fitness by decreasing an enzyme to produce collagen from the precursor thereof and thus decreasing the biosynthesis amount of collagen. Thus, the condition of the skin may be improved by the ulvan derivative of the present invention. For example, the wrinkles of the skin and the decay of skin fitness caused by aging may be improved.

The anti-aging agent of the present invention comprises the ulvan derivative as an active ingredient. The active ingredient means the component having an anti-aging effect among the components contained in the anti-aging agent of the present invention. In other words, the anti-aging agent of the present invention comprises the ulvan derivative in the amount to exert an anti-aging effect. Specifically, the concentration of the ulvan derivative in the anti-aging agent of the present invention is not particularly restricted and may be adjusted to, for example, 10 mass% or more and 100 mass% or less. When the anti-aging agent of the present invention is an external preparation, the concentration of the ulvan derivative may be adjusted to 0.1 mass% or more and 10 mass% or less.

The method for retarding senescence of an animal according to the present invention comprises the step of administering the anti-aging agent of the present invention comprising the H-type ulvan or the alkali metal ion salt of ulvan as an active ingredient to the animal. Both of the H-type ulvan and the alkali metal ion salt of ulvan may be administered, or either of the H-type ulvan or the alkali metal ion salt of ulvan may be administered.

The administration frequency and the administration dose of the anti-aging agent according to the present invention may be appropriately adjusted depending on the age, sex, condition or the like of a patient to be administered, and the amount to exert an anti-aging effect is administered to a patient. For example, the administration amount of the anti-aging agent per 1 day may be adjusted to 1 mg/kg weight or more and 1 g/kg weight or less. When the anti-aging agent is an external preparation, the application amount per 1 day may be adjusted to 0.1 mg or more and 10 mg or less. The administration frequency and the application frequency per 1 day are not particularly restricted, and the anti-aging agent may be administered or applied in a single dose or in multiple dose within the desired administration range.

The anti-aging agent of the present invention may be administered to not only a human but also an animal other than a human. An example of the objective animal to be administered includes a domestic animal such as horse, cow, pig, sheep, goat, camel and llama; a race animal such as racehorse; a pet animal such as dog and cat; an experimental animal such as mouse, rat, guinea pig and rabbit; and poultry such as chicken, duck, turkey and ostrich.

The dosage form of the anti-aging agent according to the present invention is not particularly restricted. For example, the dosage form may be the ulvan derivative itself, a composition in combination with the other component, or the solution or the suspension thereof. The dosage form of the anti-aging agent according to the present invention is not particularly restricted, and an example thereof includes tablet, powder, capsule, sugar-coated tablet, granule, liquid formulation and external preparation. A pharmaceutically acceptable additive may be used for the anti-aging agent of the present invention depending on the dosage form thereof. An example of such an additive includes excipient, disintegrant, lubricant, binder, antioxidant, colorant, sweetener, aggregation preventing agent, preservative, solubilizing agent for the active ingredient, and stabilizing agent.

The ulvan derivative of the present invention has an objective and excellent anti-aging effect such as mitochondria-activating action, antioxidant action and collagen biosynthesis-enhancing action. In addition, the ulvan derivative of the present invention may be highly safe, since the ulvan derivative is the free form or an alkali metal ion salt of the polysaccharide derived from algae. Thus, the anti-aging agent of the present invention can be continuously ingested as a health food or the like having excellent anti-aging effect.

For example, the ulvan derivative of the present invention can be used for a general drink or food as the anti-aging agent. The drink or food containing the ulvan derivative of the present invention is not particularly restricted, and an example thereof includes a drink such as milk beverage, refreshing beverage, isotonic drink, energy drink, beauty care drink and liquid nutritional supplement; a sweet such as chewing gum, chocolate, candy, jelly, cake, biscuit and cracker; a frozen dessert such as ice cream and ice; a noodle such as wheat noodle, Chinese noodle, spaghetti and instant noodle; a fish paste food such as fish cake, tube-shaped fish paste cake and pounded fish cake; a condiment such as dressing, mayonnaise and sauce; bread, ham, rice porridge, cooked rice, soup, various boil-in-the-bag meals and various frozen food. The drink and food comprising the ulvan derivative of the present invention can be used as health food, supplement, functional food, foods with functional claims, dietary supplement, food for specified health use, food with nutrient function claims, care food, smile care food, chewing and swallowing assisting food, concentrated liquid diet, food for patients or the like.

The present application claims the benefit of the priority date of Japanese patent application No. 2022-170789 filed on October 25, 2022. All of the contents of the Japanese patent application No. 2022-170789 filed on October 25, 2022, are incorporated by reference herein.

### EXAMPLES

Hereinafter, the present invention is described in more detail with Examples. The present invention is however not restricted to the following Examples in any way, and it is possible to work the present invention according to the Examples with an additional appropriate change within the range of the above descriptions and the following descriptions. Such a changed embodiment is also included in the technical scope of the present invention.

### Example 1: Production of ulvan AMI salt

### (1) Production of extracted natural ulvan

Ulva meridionalis was washed well and freeze-dried and then pulverized using a blender. Water (20 mL) was added to the obtained dried powder (1 g) for the hot water extraction at 90°C. The supernatant liquid was separated, and distilled water (20 mL) was added to the residue for the hot water extraction again. This procedure was repeated two times. The separated aqueous solution (about 60 mL) was concentrated, and then ethanol was added thereto and the ethanol concentration was adjusted to 75% to precipitate ulvan. The produced precipitate was obtained by centrifugation and freeze-dried to obtain extracted natural ulvan.

### (2) Production of ulvan AMI salt

OH-type anion-exchange resin was produced from Cl-type anion-exchange resin ("Amberlite^{™} IRA410Cl" manufactured by Organo) using an NaOH aqueous solution. The produced OH-type anion-exchange resin (0.5 g) was added to a 0.5% aqueous solution of the extracted natural ulvan (100 g), and the mixture was stirred at room temperature for 30 minutes. The supernatant liquid was obtained, new OH-type anion-exchange resin was added thereto, and the mixture was similarly treated. The treatment using the OH-type anion-exchange resin and the collection of the supernatant liquid were totally repeated 5 times to remove a sulfate salt (SO₄²⁻) and chloride ion (Cl⁻) from the aqueous solution.

A part of the cation at the cation site of ulvan was exchanged for Na ion by adding Na-type cation-exchange resin ("Amberlite^{™} IR120BNa" manufactured by Organo) (0.5 g) to the obtained aqueous ulvan solution (about 100 g), and then the mixture was freeze-dried.

The molecular weight of the obtained ulvan AMI salt was measured by gel permeation chromatography (GPC). In addition, the concentration of the cation contained in the obtained ulvan AMI salt was measured by induction coupled plasma (ICP) emission spectrometric analysis. The result is shown in Table 1. In Table 1, the concentration of the proton is an estimate value based on the ICP emission spectrometric analysis, and the "ratio of alkali metal ion (AMI) + H⁺" was calculated by calculating the acidic group concentration using the following formula from the result of the ICP emission spectrometric analysis and calculating the ratio of the alkali metal ion and H⁺ to the calculated acidic group concentration. Since the obtained ulvan Na salt hardly contained proton, the ratio of the alkali metal ion to the acidic group was regarded as 41 mol%. Acidic group concentration = (Na+ concentration + K+ concentration + H+ concentration) + 2 × (Ca2+ concentration + Mg2+ concentration)

### Example 2: Production of H-type ulvan

The OH-type anion-exchange resin (0.5 g) was added to 0.5% aqueous solution of the extracted natural ulvan (100 g), and the mixture was stirred at room temperature for 30 minutes. The supernatant liquid was obtained, new OH-type anion-exchange resin was added thereto, and the mixture was similarly treated again. The treatment with OH-type anion-exchange resin and the collection of the supernatant liquid were repeated 5 times to remove a sulfate salt (SO₄²⁻) and chloride ion (Cl⁻) from the aqueous ulvan solution.

Na-type cation-exchange resin ("Amberlite^{™} IR120BNa" manufactured by Organo) (0.5 g) was added to the obtained aqueous ulvan solution (about 100 g), and the mixture was stirred at room temperature for 30 minutes. A part of the cation at the cation site of ulvan was exchanged for H ion by adding H-type cation-exchange resin ("Amberlite^{™} IR120BH" manufactured by Organo) (0.45 g) to the supernatant liquid (90 mL) and stirring the mixture at room temperature for 30 minutes, and the ulvan was freeze-dried.

The obtained H-type ulvan was analyzed similarly to Example 1(2). The result is shown in Table 1. The ratio of the proton to the acidic group in the obtained H-type ulvan was 38 to 42 mol%, and the ratio of the alkali metal ion was 10 mol%.

### Example 3: Production of ulvan AMI

The OH-type anion-exchange resin (0.5 g) was added to 0.5% aqueous solution (100g) of the extracted natural ulvan, and the mixture was stirred at room temperature for 30 minutes. The supernatant liquid was separated, new OH-type anion-exchange resin was added thereto again, and the mixture was similarly treated. The treatment with OH-type anion-exchange resin and the collection of the supernatant liquid were totally repeated 5 times to remove a sulfate salt (SO₄²⁻) and chloride ion (Cl⁻) from the aqueous ulvan solution.

Then, H-type cation-exchange resin ("Amberlite^{™} IR120BH" manufactured by Organo) (0.5 g) was added to the obtained aqueous ulvan solution (about 100 g), and the mixture was stirred at room temperature for 30 minutes. The resin was removed, and the supernatant liquid was stirred for 30 minutes again. In addition, most of the cation at the cation site of the ulvan was exchanged for Na ion by adding Na-type cation-exchange resin ("Amberlite^{™} IR120BNa" manufactured by Organo) (47.3 g) to the supernatant liquid (94.6 mL), and then the ulvan was freeze-dried.

The obtained ulvan AMI salt was analyzed similarly to Example 1(2). The result is shown in Table 1. The ratio of the alkali metal ion (AMI) to the acidic group was regarded as 93 mol%, since the obtained ulvan Na salt hardly contained proton.

**[Table 1]**

| | Molecular weight | Cation concentration (mmol/g) | | | | | Ratio of proton | Ratio of AMI | Ratio of AMI+proton |
|---|---|---|---|---|---|---|---|---|---|
| | | Na | K | Ca | Mg | H (estimate) | | | |
| Raw material natural ulvan | About 800,000 | 0.2 | 0.4 | 0.2 | 1.0 | trace | 0mol% | 20mol% | 20mol% |
| Example 1 | About 800,000 | 1.0 | 0.1 | 0.1 | 0.7 | trace | 0mol% | 41mol% | 41mol% |
| Example 2 | About 800,000 | 0.3 | 0 | 0.1 | 0.6 | 1.0-1.2 | 38-42mol% | 10mol% | 48-52mol% |
| Example 3 | About 800,000 | 2.7 | 0 | 0.1 | 0 | trace | 0mol% | 93mol% | 93mol% |

### Example 4: Measurement of senescence beta galactosidase activity as senescence marker

The culture medium prepared by mixing 10% FBS, 100 µg/mL penicillin and 100 µg/mL streptomycin in MEMα medium was used for cultivating human skin fibroblast (NB1RGB cell) for 80 to 89 days to obtain senescence cell.

Natural ulvan, the ulvan AMI salt of Example 1, the H-type ulvan of Example 2 or the ulvan AMI salt of Example 3 was added to the above-described culture medium in a concentration of 500 µg/mL, and the human skin fibroblast was further cultivated at 37°C for 24 hours. Separately, the human skin fibroblast was similarly cultivated except that the ulvans were not added for comparison.

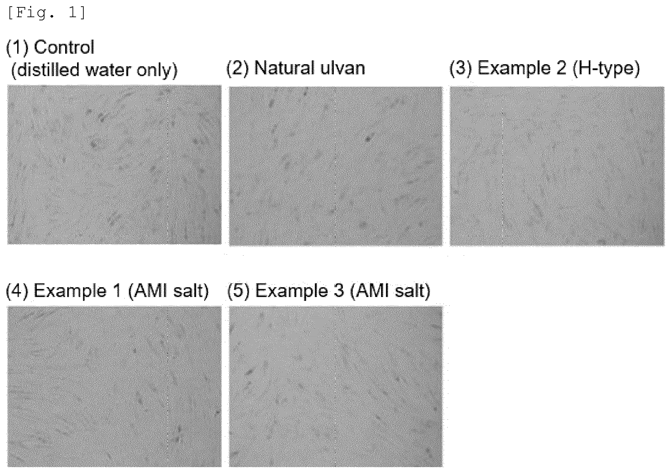

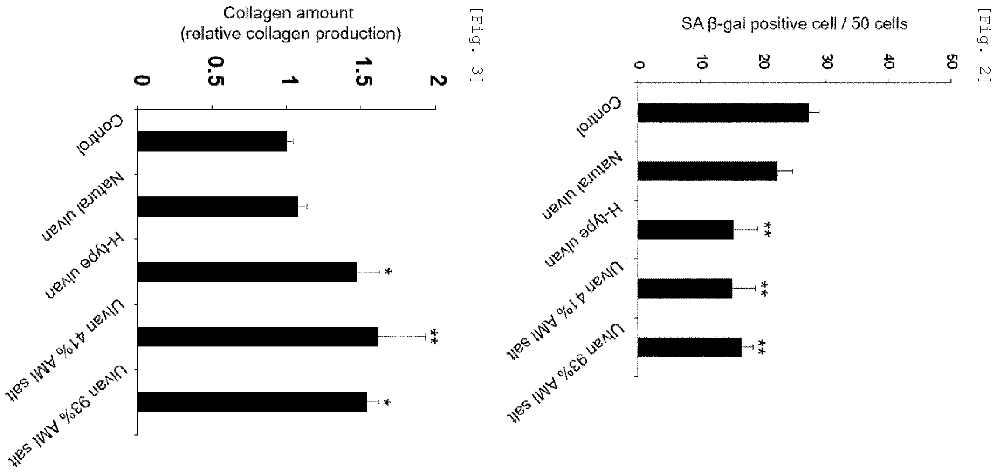

The activity of senescence beta-galactosidase as a senescence marker was measured by using Senescence Detection Kit manufactured by abcam to fix the cultivated cell with a fixative solution and stain the fixed cell in accordance with the protocol of the maker. The cell having senescence beta-galactosidase activity, i.e. senescence cell, was stained blue. The number of the cell stained blue among the cultivated 50 cells was counted. Figure 1 are enlarged photographs of the stained cells, and Figure 2 shows the number of the stained cells. In Figure 2, the "**" shows a significant difference with p<0.01 to the control group by two-way analysis of variance and subsequent Tukey's test.

Even if natural ulvan is used, the activity of senescence beta galactosidase as a senescence marker of a senescence cell cannot be significantly reduced as the results shown in Figure 1 and Figure 2.

On the one hand, it was found that the activity of senescence beta galactosidase can be significantly reduced by using the ulvan AMI salt and the H-type ulvan, in which the counter cation binding to the carboxy group is substituted with proton and/or sodium ion.

### Example 5: Collagen productivity test

The senescence human skin fibroblast was further cultivated at 37°C for 72 hours with using the culture medium prepared by adding natural ulvan, the ulvan AMI salt of Example 1, the H-type ulvan of Example 2 or the ulvan AMI salt of Example 3 in a concentration of 500 µg/mL to obtain samples. Separately, the sample was obtained by similarly cultivating the cell except that the ulvans were not added for comparison. A cell solution was prepared from the obtained sample, and the concentrations of a protein in the cell solutions were adjusted to the same. The amount of collagen in the cell per 25 µL of the cell solution was measured using collagen quantitative determination kit manufactured by Cosmo Bio in accordance with the protocol of the maker. The fluorescence was measured by fluorescence microplate reader ("Infinite M200" manufactured by TECAN) equipped with a pair of filters of 380 nm/485 nm. The result is shown in Figure 3. In Figure 3, the "*" shows a significant difference with p<0.05 to the control group by two-way analysis of variance and subsequent Tukey's test, and the "**" shows a significant difference with p<0.01 by two-way analysis of variance and subsequent Tukey's test.

Even if natural ulvan is used, the productivity of collagen, which is involved in skin fitness or the like, by a skin cell cannot be significantly increased as the result shown in Figure 3.

On the one hand, it was found that the productivity of collagen by a skin cell can be significantly increased by using the H-type ulvan and the ulvan AMI salt, in which the counter cation binding to the carboxy group is substituted with an alkali metal ion (AMI).

### Example 6: Analysis of SOD2 biosynthesis amount

The SOD2 biosynthesis amount was measured using anti-SOD2 antibody manufactured by Cell Signaling Technology and anti-β-actin manufactured by sigma.

Specifically, senescence human skin fibroblast was further cultivated at 37°C for 72 hours with using the culture medium prepared by adding the ulvan AMI salt of Example 1, the H-type ulvan of Example 2 or the ulvan AMI salt of Example 3 in a concentration of 500 µg/mL to obtain samples. A protein solution was prepared from the samples and subjected to polyacrylamide electrophoresis. Then, the protein was transferred to a membrane. An anti-SOD2 antibody was diluted by 1000 times, and anti-β-actin antibody was diluted by 10000 times. The membrane was immersed into the diluted solution, incubated at 4°C for 18 hours, and then washed. Next, an HRP-labeled anti rabbit antibody and anti mouse secondary antibody product manufactured by Promega were diluted by 5000 times, and the membrane was immersed therein. The mixture was incubated at atmospheric temperature for 1 hour, and then the membrane was washed. Next, the membrane was immersed in a luminol reaction solution, and the mixture was incubated at atmospheric temperature for 5 to 10 minutes until light was emitted, and exposed on an X-ray film to be developed.

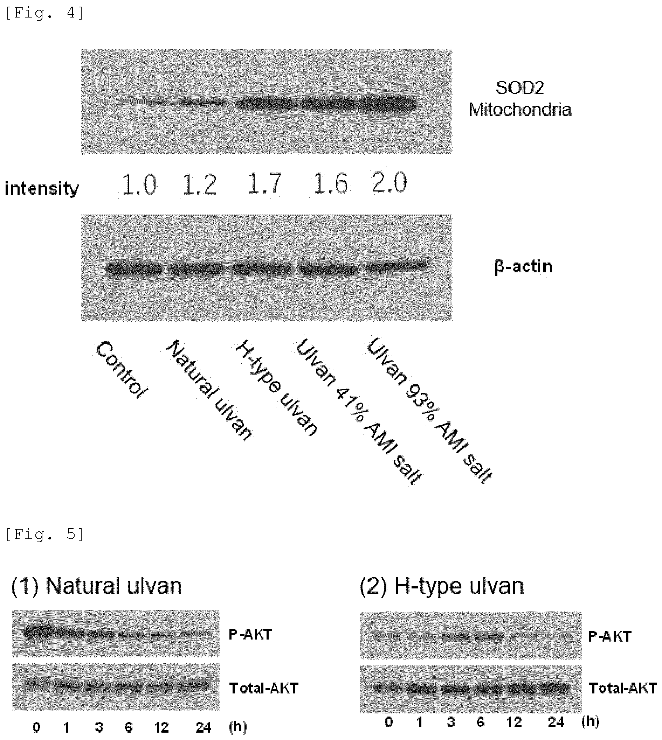

The fluorescence intensity of SOD2 band was corrected by the fluorescence intensity of β-actin band of each sample. The result is shown in Figure 4. The "Intensity" in Figure 4 is the above-described corrected value.

It was demonstrated by the result shown in Figure 4 that the biosynthesis amount of SOD2 produced in mitochondria among SOD (Superoxide dismutase) to disproportionate reactive oxygen species, which causes senescence by oxidative stress, into oxygen and hydrogen peroxide can be increased by the ulvan AMI salt and the H-type ulvan.

### Example 7: Analysis of activated (phosphorylated) AKT

The activation of serine/threonine kinase (AKT) was analyzed using anti-phosphorylated AKT antibody manufactured by Cell Signaling Technology and anti-AKT antibody manufactured by Cell Signaling Technology.

Specifically, the culture medium to which natural ulvan or the H-type ulvan was added in a concentration of 100 µg/mL was used to further cultivate senescence human skin fibroblast at 37°C for the described times, and the samples were prepared. Protein solutions were prepared from the samples, subjected to polyacrylamide electrophoresis, and then transferred to a membrane. An anti-phosphorylated AKT antibody product was diluted by 1000 times, and anti-AKT antibody was diluted by 1000 times. The membrane was immersed therein, and the mixture was incubated at 4°C for 18 hour and then the membrane was washed. Next, HRP-labelled anti rabbit secondary antibody product manufactured by Promega was diluted by 5000 times, and the membrane was immersed therein. The mixture was incubated at atmospheric temperature for 1 hour, and then the membrane was washed. Then, the membrane was immersed in a luminol reaction solution, and the mixture was incubated at atmospheric temperature for 5 to 10 minutes until light was emitted, and exposed on an X-ray film to be developed. The result is shown in Figure 5.

The H-type ulvan may accelerate the cell growth decreased by senescence, since the activation of AKT accelerates cell growth. In addition, this effect can be obtained all the more by the H-type ulvan, since natural ulvan cannot activate AKT.

### Example 8: Mitochondria activation test

The senescence human skin fibroblast was further cultivated at 37°C for 24 hours with using the culture medium prepared by adding natural ulvan, the ulvan AMI salt of Example 1, the H-type ulvan of Example 2 or the ulvan AMI salt of Example 3 in a concentration of 500 µg/mL to the above-described medium. Separately, the senescence human skin fibroblast was similarly cultivated except that the ulvans were not added for comparison.

Then, the activity of mitochondria was measured by TMRM staining with using mitochondria membrane potential detection reagent ("TMRM (tetramethylrhodamine methyl ester)" manufactured by Thermo) in accordance with the protocol of the maker. Fluorescence was measured using a pair of filters of 535 nm/590 nm and fluorescence microplate reader ("Infinite M200" manufactured by TECAN).

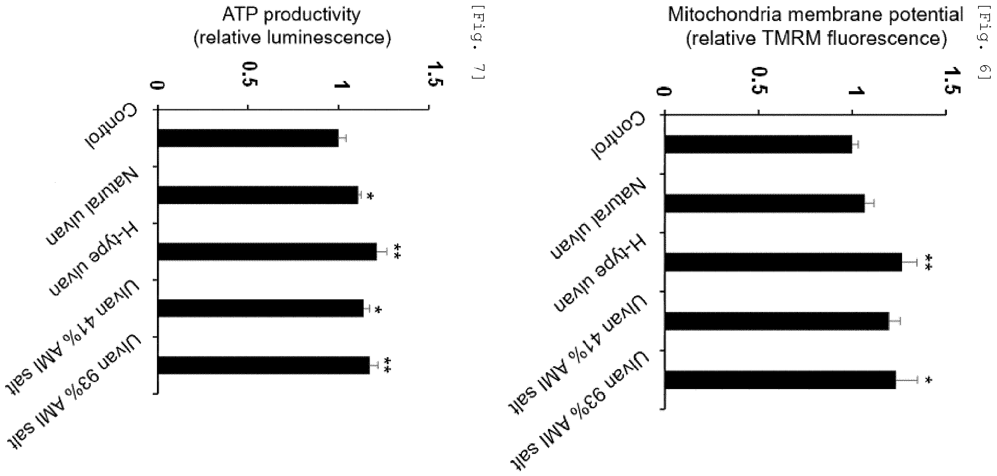

When the activity of mitochondria is high, membrane potential difference is maintained, TMRM as a low molecular fluorescent dye aggregates and thus stronger fluorescence is emitted. When the activity is decreased, membrane potential difference is decreased, TMRM does not aggregate and thus weaker fluorescence is emitted. The mitochondria activity, therefore, can be measured as fluorescent intensity of the wavelength measured by 535 nm/590 nm. The result is shown in Figure 6 as the ratio of the embodiment treated with ulvan and the like to the control example. In Figure 6, the "*" shows a significant difference with p<0.05 by two-way analysis of variance and subsequent Tukey's test, and the "**" shows a significant difference with p<0.01 by two-way analysis of variance and subsequent Tukey's test.

It was found from the result shown in Figure 6 that the H-type ulvan and the ulvan AMI salt can significantly improve the activity of mitochondria, which produces energy and therefore relates to the activity of a cell.

### Example 9: Production amount of ATP

The senescence human skin fibroblast was further cultivated at 37°C for 24 hours with using the culture medium prepared by adding natural ulvan, the ulvan AMI salt of Example 1, the H-type ulvan of Example 2 or the ulvan AMI salt of Example 3 in a concentration of 500 µg/mL to obtain samples. Separately, the senescence human skin fibroblast was similarly cultivated except that the ulvans were not added for comparison. Then, the production amount of ATP was measured using CellTiter-Glo (registered trademark) 2.0 Assay manufactured by Promega in accordance with the protocol of the maker. Light emitting was measured by Promega GloMax 20/20 Luminometer. The result is shown in Figure 7 as the ratio of the embodiment treated with ulvan and the like to the control example. The "*" shows a significant difference with p<0.05 by two-way analysis of variance and subsequent Tukey's test, and the "**" shows a significant difference with p<0.01 by two-way analysis of variance and subsequent Tukey's test.

It was found from the result shown in Figure 7 that natural ulvan and ulvan 41 mol% AMI salt can significantly increase the production amount of ATP, which an energy transmitter for chemical reactions within a living body at a skin cell, and the H-type ulvan and ulvan 93 mol% AMI salt can further increase the production amount of ATP.

### Example 10: Measurement of antioxidant activity

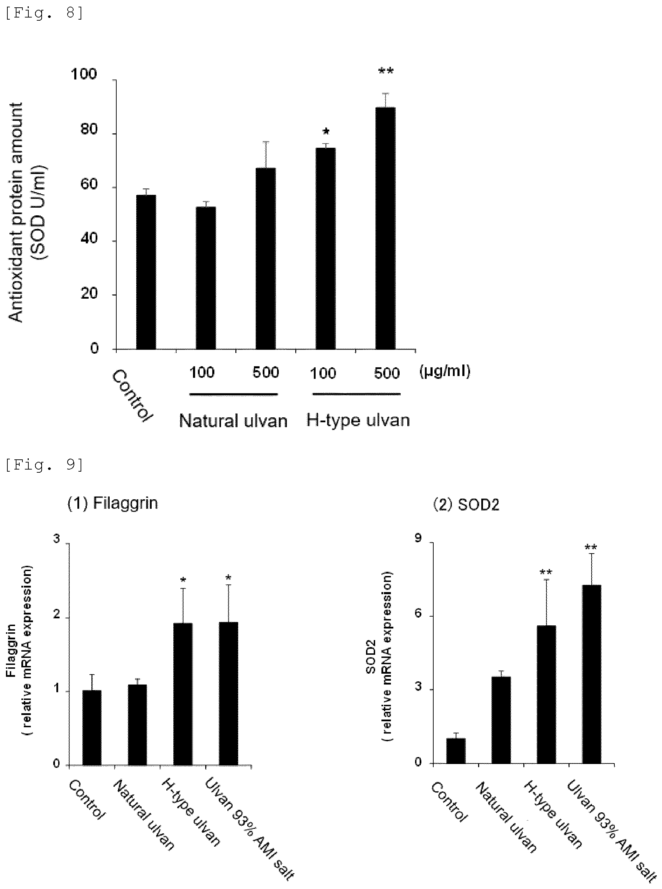

The senescence human skin fibroblast was further cultivated at 37°C for 48 hours with using the culture medium prepared by adding natural ulvan or the ulvan H in a concentration described in Figure 8 to obtain samples. Separately, the senescence human skin fibroblast was similarly cultivated except that the ulvans were not added for comparison to obtain a sample.

The antioxidant activity of the obtained samples was measured using an antioxidant ability measurement kit ("SOD Assay Kit-WST" manufactured by Dojindo Molecular Technologies) in accordance with the protocol of the maker. The fluorescence was measured by fluorescence microplate reader ("Infinite M200" manufactured by TECAN) equipped with a pair of filters of 380 nm/485 nm. The result is shown in Figure 8. In Figure 8, the "*" shows a significant difference with p<0.05 by two-way analysis of variance and subsequent Tukey's test, and the "**" shows a significant difference with p<0.01 by two-way analysis of variance and subsequent Tukey's test.

It was demonstrated as the result shown in Figure 8 that natural ulvan cannot significantly increase an antioxidant protein amount but the H-type ulvan can significantly increase an antioxidant protein amount.

### Example 11: Analysis of mRNA expression

The human epidermal keratinocyte (NHEK cell (AD: adult)) manufactured by Kurabo Industries was further cultivated at 37°C for 24 hours in the culture medium to which ulvan, the H-type ulvan or ulvan 93 mol% AMI salt was added in a concentration of 500 µg/mL. The sample was obtained 24 hours after the start of the cultivation, and the gene expression levels of Filaggrin as an epidermal protective factor and superoxide dismutase 2 (SOD2) as an antioxidant enzyme of mitochondria were determined using a highly efficient Master Mix for real-time PCR ("THUNDERBIRD (registered trademark) Next SYBR (registered trademark) qPCR Mix" manufactured by TOYOBO). The total RNA was normalized in each reaction using β-actin complementary DNA as an internal standard. The result is shown in Figure 9. In Figure 9, the "*" shows a significant difference with p<0.05 by two-way analysis of variance and subsequent Tukey's test, and the "**" shows a significant difference with p<0.01 by two-way analysis of variance and subsequent Tukey's test.

It was demonstrated as the result shown in Figure 9 that natural ulvan cannot significantly accelerate the expressions of Filaggrin as an epidermal protective factor and SOD2 as an antioxidant enzyme of mitochondria but the H-type ulvan and ulvan 93 mol% AMI salt can significantly increase the expressions of Filaggrin and SOD2.

### Example 12: Production H-type ulvan

The H-type ulvan of which alkali metal ion ratio to the acidic group was 19 to 21 mol% and of which alkali metal ion ratio was 15 mol% was produced by mixing natural ulvan of Example 1(1) and the H-type ulvan (H⁺ ratio: 38 to 42 mol%).

### Example 13: Production of ulvan AMI salt

The ulvan AMI salt of which alkali metal ion ratio to the acidic group was 30 mol% was prepared by mixing natural ulvan of Example 1(1) and the ulvan AMI salt (AMI ratio: 41 mol%).

**[Table 2]**

| | Cation concentration (mmol/g) | | | | | Ratio of proton | Ratio of AMI | Ratio of AMI+proton |
|---|---|---|---|---|---|---|---|---|
| | Na | K | Ca | Mg | H (estimate) | | | |
| Raw material natural ulvan | 0.2 | 0.4 | 0.2 | 1.0 | trace | 0mol% | 20mol% | 20mol% |
| Example 12 | 0.2 | 0.2 | 0.1 | 0.8 | 0.5-0.6 | 19-21mol% | 15mol% | 34-36mol% |
| Example 13 | 0.6 | 0.2 | 0.1 | 0.8 | trace | 0mol% | 30mol% | 30mol% |

### Example 14: Analysis of mRNA expression

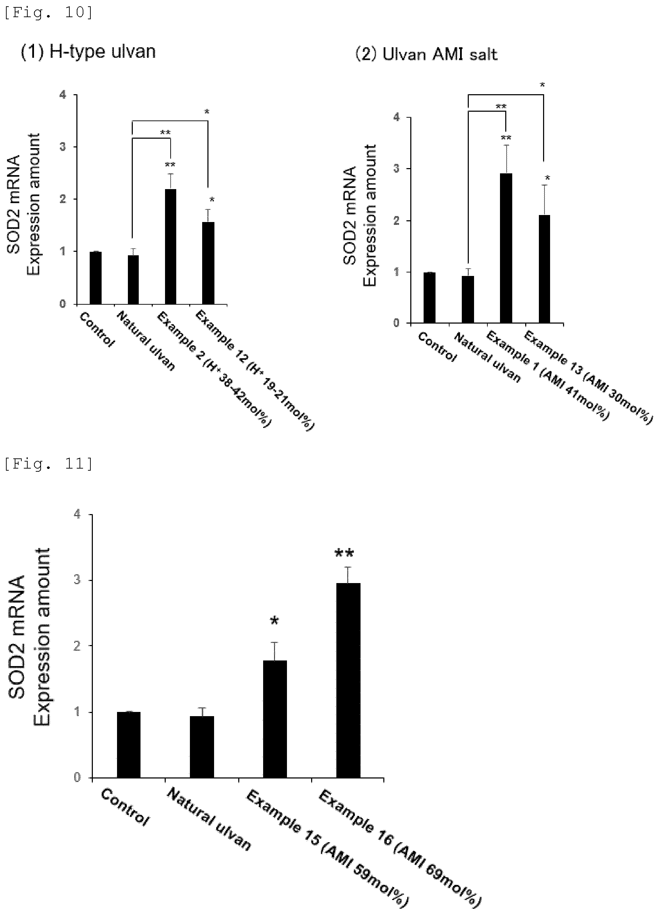

The gene expression amount of superoxide dismutase 2 (SOD2) derived from the human epidermal keratinocyte in the culture media to which natural ulvan, the H-type ulvan (H⁺ ratio: 38 to 42 mol%) of Example 2, the H-type ulvan (H⁺ ratio: 19 to 21 mol%) of Example 12, the ulvan AMI salt (AMI ratio: 41 mol%) of Example 1 and the ulvan AMI salt (AMI ratio: 30 mol%) of Example 13 were added was determined similarly to Example 11. The result of the H-type ulvan is shown in Figure 10(1), and the result of the ulvan AMI salt is shown in Figure 10(2). In Figure 10, the "*" shows a significant difference with p<0.05 by two-way analysis of variance and subsequent Tukey's test, and the "**" shows a significant difference with p<0.01 by two-way analysis of variance and subsequent Tukey's test.

It was demonstrated as the result shown in Figure 10 that natural ulvan cannot significantly accelerate the expression of SOD2 as an antioxidant of mitochondria but even the H-type ulvan having a relatively low proton ratio and the ulvan AMI salt having a relatively low AMI ratio can significantly increase the expression of SOD2.

### Example 15: Production of K-type ulvan AMI salt

H-type cation-exchange resin ("Amberlite^{™} IR120BH" manufactured by Organo, 0.5 g) was added to the aqueous ulvan solution (about 100 g) obtained similarly to Example 1(2), and the mixture was stirred at room temperature for 30 minutes. The resin was removed, and the remaining solution was stirred again for 30 minutes. Separately, K-type cation-exchange resin was prepared by exchanging the H⁺ of H-type cation-exchange resin ("Amberlite^{™} IR120BH" manufactured by Organo) for K⁺ with using potassium nitrate. The K-type cation-exchange resin (1.0 g) was added to the aqueous ulvan solution (98 mL) from which the resin had been removed, and the mixture was stirred at room temperature for 30 minutes to exchange the cation at the cation site of the ulvan for potassium ion. The resin was removed, and the remaining solution was stirred again for 30 minutes. Then, the mixture was freeze-dried to obtain K-type ulvan AMI salt.

The produced K-type ulvan AMI salt was analyzed similarly to Example 1(2); as a result, the ratio of the alkali metal ion to the acidic group was 59 mol%. The analytical result is shown in Table 3.

### Example 16: Production of K-type ulvan AMI salt

A K-type ulvan AMI salt was produced similarly to Example 12 except that the usage amount of the K-type cation-exchange resin was changed from 1.0 g to 2.5 g.

The produced K-type ulvan AMI salt was analyzed similarly to Example 1(2); as a result, the ratio of the alkali metal ion to the acidic group was 69 mol%. The analytical result is shown in Table 3.

**[Table 3]**

| | Cation concentration (mmol/g) | | | | | Ratio of proton | Ratio of AMI | Ratio of AMI+proton |
|---|---|---|---|---|---|---|---|---|
| | Na | K | Ca | Mg | H (estimate) | | | |
| Raw material natural ulvan | 0.2 | 0.4 | 0.2 | 1.0 | trace | 0mol% | 20mol% | 20mol% |
| Example 15 | 0.1 | 1.6 | 0.1 | 0.5 | trace | 0mol% | 59mol% | 59mol% |
| Example 16 | 0 | 1.8 | 0.1 | 0.3 | trace | 0mol% | 69mol% | 69mol% |

### Example 17: Analysis of mRNA expression

The amount of the gene expression of superoxide dismutase 2 (SOD2) derived from the human epidermal keratinocyte in the culture media to which natural ulvan and the K-type ulvan AMI salts of Examples 15 and 16 were added was determined similarly to Example 11. The result is shown in Figure 11. In Figure 11, the "*" shows a significant difference with p<0.05 by two-way analysis of variance and subsequent Tukey's test, and the "**" shows a significant difference with p<0.01 by two-way analysis of variance and subsequent Tukey's test.

It was demonstrated as the result shown in Figure 11 that the ulvan AMI salt having relatively large amount of potassium ion can also significantly increase the expression of SOD2, and the larger the ratio of AMI is, the higher the effect is.

## Claims

1. An anti-aging agent comprising an H-type ulvan or an alkali metal ion salt of ulvan as an active ingredient.

2. The anti-aging agent according to claim 1, wherein the alkali metal is Na and/or K.

3. The anti-aging agent according to claim 1, wherein 15 mol% or more of a carboxy group and a sulfate group of the H-type ulvan is protonated.

4. The anti-aging agent according to claim 1, wherein 30 mol% or more of a carboxy group and a sulfate group of the alkali metal ion salt of ulvan form the salt of the alkali metal ion.

5. The anti-aging agent according to claim 1, having a mitochondria activating action.

6. The anti-aging agent according to claim 1, having an antioxidant action.

7. The anti-aging agent according to claim 1, having a collagen biosynthesis enhancing action.

8. Use of an H-type ulvan or an alkali metal ion salt of ulvan for retarding senescence.

9. The use according to claim 8, wherein the alkali metal is Na and/or K.

10. The use according to claim 8, wherein 15 mol% or more of a carboxy group and a sulfate group of the H-type ulvan is protonated.

11. The use according to claim 8, wherein 30 mol% or more of a carboxy group and a sulfate group of the alkali metal ion salt of ulvan form the salt of the alkali metal ion.

12. The use according to claim 8, wherein senescence is retarded by activating mitochondria.

13. The use according to claim 8, wherein senescence is retarded by an antioxidant action.

14. The use according to claim 8, wherein senescence is retarded by enhancing collagen biosynthesis.

15. A method for retarding senescence of an animal, comprising the step of administering an H-type ulvan or an alkali metal ion salt of ulvan to the animal.

16. The method according to claim 15, wherein the alkali metal is Na and/or K.

17. The method according to claim 15, wherein 15 mol% or more of a carboxy group and a sulfate group of the H-type ulvan is protonated.

18. The method according to claim 15, wherein 30 mol% or more of a carboxy group and a sulfate group of the alkali metal ion salt of ulvan form the salt of the alkali metal ion.

19. The method according to claim 15, wherein the animal is a human.

20. The method according to claim 15, wherein the animal is an animal other than a human.

21. The method according to claim 15, wherein senescence is retarded by activating mitochondria.

22. The method according to claim 15, wherein senescence is retarded by an antioxidant action.

23. The method according to claim 15, wherein senescence is retarded by enhancing collagen biosynthesis.
